(19)

Europäisches Patentamt
European Patent Office
Office européen des brevets

(11) **EP 2 932 261 B1**

(12) **EUROPEAN PATENT SPECIFICATION**

(45) Date of publication and mention
of the grant of the patent:
**17.01.2024 Bulletin 2024/03**

(21) Application number: **13824511.3**

(22) Date of filing: **03.12.2013**

(51) International Patent Classification (IPC):
*G01N 33/49* *(2006.01)*    *G01N 33/86* *(2006.01)*

(52) Cooperative Patent Classification (CPC):
**G01N 33/4905**

(86) International application number:
**PCT/HU2013/000117**

(87) International publication number:
**WO 2014/091262 (19.06.2014 Gazette 2014/25)**

(54) **DISPOSABLE IVD DEVICE FOR HOME SELF-CHECKING OF INR VALUE, CHARACTERISING BLOOD CLOTTING**

EINWEG-IVD-VORRICHTUNG ZUR SELBSTPRÜFUNG DES DIE BLUTGERINNUNG KENNZEICHNENDEN INR-WERTES

DISPOSITIF DE GOUTTE-À-GOUTTE INTRAVEINEUX JETABLE POUR AUTOCONTRÔLE À DOMICILE DE VALEUR INR, CARACTÉRISANT LA COAGULATION SANGUINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.12.2012 HU P1200732**

(43) Date of publication of application:
**21.10.2015 Bulletin 2015/43**

(73) Proprietor: **Haemothromb Test Kft.**
**3360 Heves (HU)**

(72) Inventor: **SUBOTICS, Gyula**
**9022 Györ (HU)**

(74) Representative: **Szabo, Zsolt**
**Danubia**
**Patent & Law Office LLC**
**Bajcsy-Zsilinszky út 16**
**1051 Budapest (HU)**

(56) References cited:
EP-A1- 2 040 073          WO-A1-96/00395
WO-A1-98/32389          US-A- 3 951 606
US-A1- 2002 187 071

## Description

The subject of the invention is a disposable IVD device for the home self-checking of INR value characterising coagulation, for measuring coagulation distance characterising blood dotting with no reagent/reagents, preferably for determining INR value, by means of blood absorbing material, with a device.

US6759009 (Wai Tak Hoorestown, Nj 6 May 2002) horizontally operated disposable device where a reagent gets in reaction with a biological fluid flowing in the capillary tube due to which a coloured congealment is formed. Prothronbin time can be read from the scale next to the coloured congealment stripe. WO9406007 (Gradipore Limited 17 March 1994), WO9832389A1 (Akzo Nobel N.V. 30 July 1998) each of these patent documents describes a method and apparatus for measuring coagulation time, where blood sample and some porous material are brought into contact, where due to capillary effects the blood sample spreads from the starting point concentrically (WO9406007), or lineally (WO9600395, WO9832389A1). The further spread of the sample stops due to coagulation. The degree of spread is proportional to coagulation time. In WO9832389A1 the distance covered by the serum after coagulation is measured after they have proved that it is proportional to coagulation time. The distance covered by the sample is determined in different ways, among others with simple visual observation. Each device has a design which assures application at the place of care e.g. at home.

In US6759009 a device carrying a similar principle into practice is described, which is different from the former ones as blood sample does not flow in a porous material during measuring, but in a capillary tube lined with a porous material and the porous material is there to help the clot get fixed.

In the case of the above described devices blood or blood plasma are mixed with a proper activator: in case of measuring prothrombine times with prothrombine or with some stuff, equivalent to it, in case of measuring thromboplastine, with thromboplastine etc. In some cases the measuring stripe can contain other types of blood clotting proteins too, which are there to improve the accuracy of measuring or to identify various coagulation disorders.

The device in US3951606 can be considered as the prototype of a disposable device, simplified to the utmost. It comprises a single capillary tube with a defined bore, where the blood sample trickling down its wall covers a distance before coagulation which is proportional to coagulation time. The result can be read from the scale on the wall of the capillary tube. Blood is brought into contact with the proper activator at the entrance of the capillary tube. There are very strict criteria of the authorization of putting devices for measuring coagulation on the market, among others e.g. that during minimum 100 measurements no false measuring result can occur, or the faulty result must be indicated by the device in each case. Due to this fact nobody has succeeded in developing a simple device which could be mass produced in a cheap way and would comply with the above regulations. Consequently there are no such devices on the market despite the fact that there is a growing demand for purchasing measuring devices for home use on a world scale.

Our objective was to develop a device beneficially for fast determination of INR value, characterising blood clotting, which eliminates the above disadvantages, indicates the false result in each case, cheap and simple structured, whose production does not require any complicated technical operations, which is easily applicable and manageable, requires little blood sample for measuring, can be mass-produced and -traded, can reliably be used without any special expertise by assuring the process of coagulation, without affecting the process artificially, without the application of any reagents. For the sake of the easy evaluation of coagulation value by patients we have supplied the measuring device with different coloured signs e.g. red for bad, light blue for still acceptable, blue and green for good values.

We came to the conclusion during our work that we could hit the target in the simplest way if we used a device comprising a measuring stripe consisting of at least two layers made by us the upper layer of which was made of absorbent material e.g. paper made of loose structured, veil-like celluloid fibres, the lower layer of which comprising thicker, solid structured cardboard with glassy and smooth surface made of less absorbent celluloid base material. The two layers were joined by sticking at least at their ends. In case of this design we have not experienced any failures of the operation of the device while observing the coagulation of several blood samples, which can be explained by the formation an operation of a multi-layered capillary tube system on the edge surface of the dual blood absorbent layer contiguous to each-other, as the role of each blocked capillary tube can be taken over by several other capillary tubes at the same time, by which the continuous blood absorption is always assured until blood clotting. In case of this design of the measuring stripe we have experienced with certain blood samples though that absorption of blood serum continued accompanied by a dim discoloration after blood absorption which did not affect the length of blood column until coagulation, yet we judged that it could be a disturbing factor for a non-expert while determining INR value from the length of blood column.

In case of the three-layered measuring stripe design the upper layer is made of absorbent material e.g. veil comprising loose-structured celluloid fibres and the two lower layers are made of thicker, solid-structured cardboard with glassy and smooth surface made of less absorbent celluloid base material. The two upper layers are still joined by sticking at their end as long as 5-5 mm so that blood absorption can continue without any obstacles in each layer despite sticking. This kind of sticking can be carried out by e.g. applying two-side self-adhesive tape of proper length. The third, lowest layer is fixed to the two upper layers so that it can slip e.g. in the way that its lower end over the reservoir is stuck to the lower, less absorbent layer of the two upper layers and its upper end is on the one hand 5-7 mm shorter than the upper layers and it touches them without any fixing so that it can slip, by which the appearance of blood serum after the end of blood clotting can be eliminated in the upper vail, comprising celluloid fibres, on the other hand the so created three-layered structure is rigid enough to prevent the occurrence of curved bendings thus preventing the occasional dislayering, eliminating the blockage of blood absorption.

In case of the four-layered measuring stripe design both the upper layer and the lower layer are made of at least two layers each, by which the error places and lack of fibres can be eliminated.

The measuring stripe is mounted in the device in a way so that its continuous contact with the device both along the lower surface and along the upper one can be avoided i.e. the contact places with the device are separated from one-another and either point-like or section-like, moreover the contact places along the lower surface as well as the upper one never overlap one-another. As the capillarity created by the special homogeneity of the measuring stripe's material structure must not be disturbed by the different capillarity emerging along the contact surface of the device of a material structure, different from that of the measuring stripe, the uniform conditions of blood absorption in the measuring stripe which are necessary for measuring can be ensured this way.

The measuring stripe can be mounted in the device in the most expedient way if we place it between the distance pieces, supplied with shoulders, protruding from the lower plane of the device at proper distances. When we close the upper plane to the lower one, the measuring stripe is fixed to the device by the distance mandrels, protruding from the upper plane. As the shoulders and distance mandrels are for keeping the measuring stripe level and in the given position in the device, the free movement of the three-layered measuring stripe's lowest layer from the two upper layers is ensured by creating a bracing slot in the flange of the device. The measures of the bracing slot (its width, length and depth) exceed the measures of the measuring stripe's crosssection by at least 0.1 mm, assuring that the extension of the measuring stripe due to the damp during coagulation could be equalized by moving in the bracing slot without any creases or dislayering.

In case of sparsely located distance pieces the bending down of the end of the measuring seripe's-lowest, i.e. third layer from the two upper layers can be eliminated by mounting in an insertion holding end.

In light of the above, our objective was achieved, in general, by constructing a disposable device for home measuring of clotting capacity of a blood sample, in particular of the INR value as defined by claim 1.

That is, the invention is a disposable device for home measuring of INR value, characterising blood clotting, moreover in case of regular modification of blood clotting capacity in an artificial way, e.g. taking medicine/medicines, for continuous monitoring of the induced change, e.g. in case of taking anticoagulants for determining INR value (International Normalized Ratio, prothrombine time patient/prothrombine time control) with blood absorbing material, with a device for measuring blood clotting distance comprising a measuring stripe brought into contact with blood sample in the reservoir, attached to the lower plane and braced between the distance mandrels and distance pieces protruding from the two at least partly transparent planes: the lower plane and the upper plane. The device comprises an expediently chosen evaluation part which is suitable for reading the boundary line of the clot on the measuring stripe off the INR value scale it is supplied with, in case of measuring the modification of the INR value of blood. The lower plane and the upper plane, bracing the measuring stripe are arranged at $0 < \alpha < 90°$ angles to horizontal line.

Preferred further embodiments of the disposable device are set forth in claims 2 to 7.

In particular, in an advantageous design the upper plane is transparent in at least one third of its length from the end opposite the reservoir.

In another advantageous design the distance pieces protruding from the lower plane are created shoulder-like.

In a further advantageous design the reservoir is supplied with a small sheet showing blood level and the level of the amount of the necessary blood is marked with a line showing blood level.

An important attribute of the invention is that the measuring stripe comprises at least two parts, the upper layer and the lower layer. The upper layer comprises absorbent material/materials e.g. paper, made of loose structured veil-like celluloid fibres, blotting paper, etc. The lower layer comprises thicker, solid structured cardboard/cardboards with glossy and smooth surface, made of less absorbent celluloid base material.

The two parts, the upper layer and at least one lower layer are joined by sticking at their ends. At least one more lower layer is stuck to the layers, fastened together at their ends in the way that the end of the stuck lower layer is placed at least 10 mm farther from the end of the measuring stripe facing the reservoir. Its upper end is at least 3 mm shorter than the end of the fastened layers placed over it. The free end of this lower layer is supported by a distant piece or an insertion holding end, fixed to the end of the measuring stripe so that the end of the lower layer can slip.

Further important attributes include that

     a.) Upper layer is loose structured veil comprising uniformly distributed fibres, ironed together with a weigh of 50-100 gr/m$^2$ and thickness of < 0.5 mm,
     b.) Lower layer/layers is/are solid structured, glossy and smooth surfaced, less absorbent cardboard/cardboards, made of celluloid base material free of wood fibres with a weight of > 100 gr/m$^2$ and thickness of 0.2-1 mm,

c.) Upper layer/layers and lower layer/layers are joined together at least at one end,

d.) Width is minimum 1 mm.

Due to the capillary effect by the division of the blood platelets in the blood sample, by the promotion of the natural blood clotting process, by bringing blood and the air betwen the celluloid fibres and the glossy, smooth surfaced cardboard into contact, the blood clotting process is accelerated. Moreover thanks to the fact that between the edge surfaces of the veil, comprising celluloid fibres and the glossy, smooth surfaced cardboard there are no error places, hindering blood absorption, it gets uniform and can be evaluated. The blood stripe, absorbed and clotted in the measuring stripe's upper layer comprising celluloid fibres, ends in a clearly visible contour in each case. Due to this phenomenon the relevant blood coagulation distances can be identified well with the calibration of the evaluation unit, placed next to the measuring stripe e.g. with the scale, suitable for determining INR value. Of course, if we change the quality of the material of the vail, applied in the upper layer of the measuring stripe, or that of the glossy, smooth surfaced cardboard in the lower layer, the character of blood absorption and the blood clotting distance will also change. The demand of the device for blood sample can also be affected by choosing the width of the measuring stripe and the composition of the material in the right way, which is very important from the point of view of the evaluation of the received blood clotting values, as, if the measuring stripe absorbs too much blood and you run out of blood from the reservoir during measurement, the device will indicate a faulty measuring result. That is why the capacity of the reservoir always must be planned so that it can be suitable for accommodating the amount of blood sample necessary for measuring the blood, owning the highest INR value on the INR scale in the evaluation unit.

One of the essential options for preventing thromboembolic diseases is oral anticoagulant therapy with coumarin derivatives in cate of mechanical artificial heart valve, auricular fibrillation or thromboembolic diseases the efficiency of long-lasting therapy is backed up by several years of experience. In spite of its proved efficiency, oral anticoagulant therapy has some risks too. Due to the differences in dosage-effect relationship and narrow therapeutic field of oral anticoagulants, it is necessary to check blood clotting regularly to prevent thromboembolism owing to underdosage and bleeding because of overdosage.

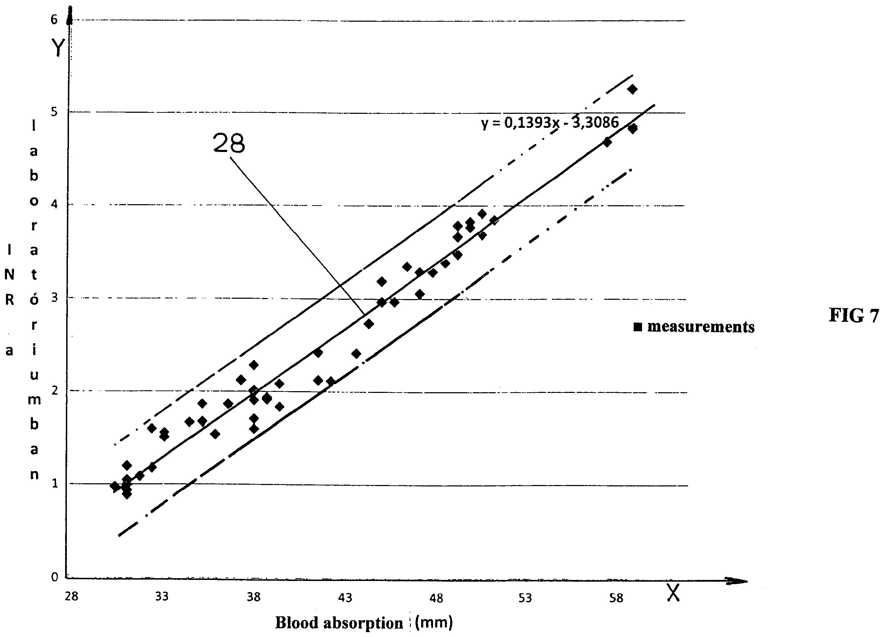

During the blood clotting examination carried out on 113 patients with doctoral cooperation, using the disposable device according to the invention, developed for rapid, home, self-checking of blood clotting, comparative laboratory examinations of blood clotting were also carried out at the same time. In case of each patient we measured the blood clotting length of the blood, absorbed in the measuring stripe of the device in millimetre and in parallel with this in the laboratory we also determined the INR value of the blood sample taken from the vein. The correlation between the INR values measured by the developed device and the INR values, measured in the laboratory are excellent (correlation factor: 0.985) According to statistical calculations the relationship between the blood clotting lengths in mm (X) in the measuring stripe of the developed device and the laboratory INR values (Y) can be characterized by

$$Y = 0.1393X\text{-}3.3085$$

regressive straight line. If we plot the results of measuring on a diagram, we can see, that the regressive straight line fits in the measured data very well. (Fig.7)

We can calculate from the equation that INR=1 value belongs to the 30.9 mm long blood clotting length of the measuring stripe.

The successive scale points of the scale, marking the INR values of the device are at a distance of 4.18 mm from each other. Possessing the calculated data, we have the opportunity to compile the INR scale, which is one of the most important parts of the device. The production of scale stickers is based on it. Moreover it makes the positioning of the scale stickers on the device possible, by which the start of the mass-production of the device is based on science.

Some preferred embodiments of the invention will be described hereinafter in details in conjunction with the accompanying drawings, wherein

FIG. 1 shows a side view of the device according to the invention in section,
FIG. 2 shows a plan view of the open device according to FIG. 1,
FIG. 3 shows a side view of a three-layered measuring stripe, supplied with the insertion holding end,
FIG. 4 shows a side view of the three-layered measuring stripe without the insertion holding end,
FIG. 5 shows a side view of the device containing the three-layered measuring stripe without the insertion holding end in section,
FIG. 6 shows a side view of the device containing the three-layered measuring stripe supplied with the insertion holding end in section,
FIG. 7 shows a graph depicting the INR values (axis Y) and the blood clotting lengths (axis X) measured in mm on the measuring stripe.

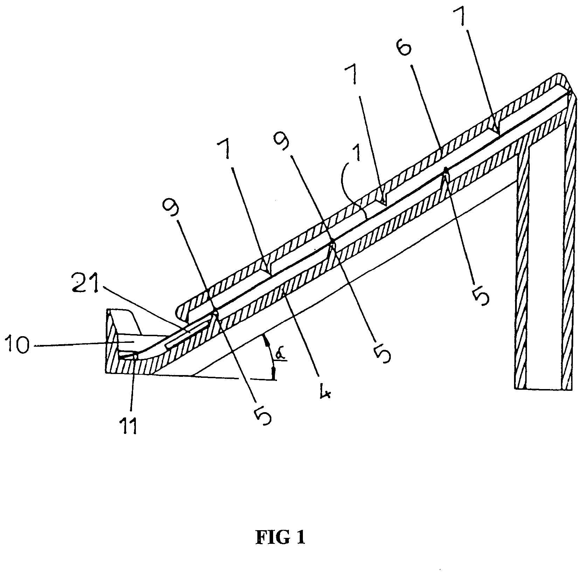

FIG. 1-2 show that the 1 measuring stripe comprises two layers: 2 upper layer and 3 lower layer. The layers are braced between the 5 distance pieces, protruding from the 4 lower plane and the 7 distance mandrels, protruding from the 6 upper plane. The 9 shoulders of the 5 distance pieces prevent the 1 measuring stripe from going into pieces and keep the 1 measuring stripe in a given position. The veil, forming the e.g. 2 upper layer of the multi-layered 1 measuring stripe and the glossy, smooth surfaced cardboard, forming the 3 lower layer never touch the 4 lower plane and the 6 upper plane except for the 5 distance piece and the 7 distance mandrel. This way it ensures the even absorption of blood in the 1 measuring stripe and prevents blood from flowing away in every direction on the surface of the 4 lower plane and of the 6 upper plane, altering the blood clotting distance, belonging to the blood sample and making it impossible to be assessed. There is a 10 reservoir on the transparent end of the 4 lower plane. The end of the 1 measuring stripe is braced by the 11 protruding pieces, protruding from the bottom of the 10 reservoir and keeping it in the given position. Finally, in an assembled state the 1 measuring stripe, the 4 lower plane and the 6 upper plane are rectangleshaped. The 10 reservoir is created at the bottom of the 4 lower plane, i.e. in one piece. A 21 small sheet showing blood level made of cardboard with a water repellent surface is built in the upper part of the 10 reservoir. The two planes comprising the 4 lower plane and the 6 upper plane are arranged at angle to the horizontal line where in case of the devices produced during development a angle was advantageously planned to be 30° as shown in FIG. 1. In case of applying a 1 measuring stripe of proper quality, this embodiment assures that during absorption there is always enough blood sample the amount of which is equal to the one, originally filled in, to be brought into contact with the 1 measuring stripe. At the lower part a 21 small sheet showing blood level supplied with a 22 line showing blood level ensures that blood absorption in the measuring stripe always starts from the same blood level and there is always enough blood available for measuring. Opening and closing the 20 plane and the 6 upper plane are ensured by 8 hinges.

The 12 flange of the 6 upper plane is supplied with 13 lock pins which become pressed in the 14 undercuts when closing the 6 upper plane on the 4 lower plane fixing them.

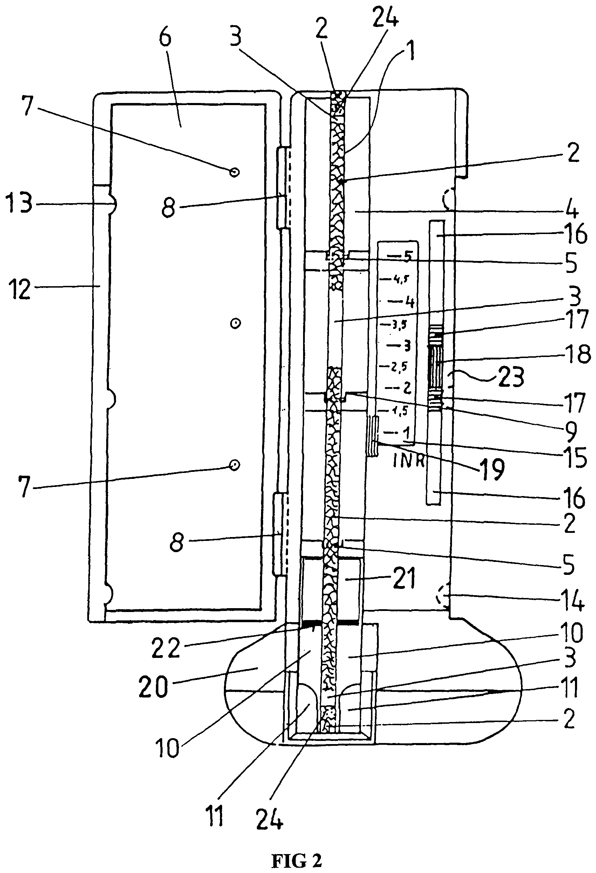

The 23 evaluation part, fixed parallel with the 1 measuring stripe is placed on the 4 lower plane and supplied with a given e.g. 15 INR value scale as shown in FIG. 2. There are also coloured signs belonging to the 23 evaluation part, placed parallel with the 1 measuring stripe which advantageously show with red, light blue, dark blue and red colours, whether the blood clotting of the patient taking an anticoagulant is well-or badly-set. If the top of the blood column, absorbed in the measuring stripe is in the 16 red zone next to the lower end of the 10 reservoir, blood clotting is badly set, there is a risk of thrombosis. If the top of the blood column is in the 17 light blue zone, blood clotting is still acceptably set. If the top of the blood column is in the 18 dark blue zone, blood clotting is properly set. If the top of the blood column is in the top 16 red zone, blood clotting is badly set again, there is a risk of bleeding. In case of people, not taking any anticoagulants the top of the blood column is in the 19 green zone.

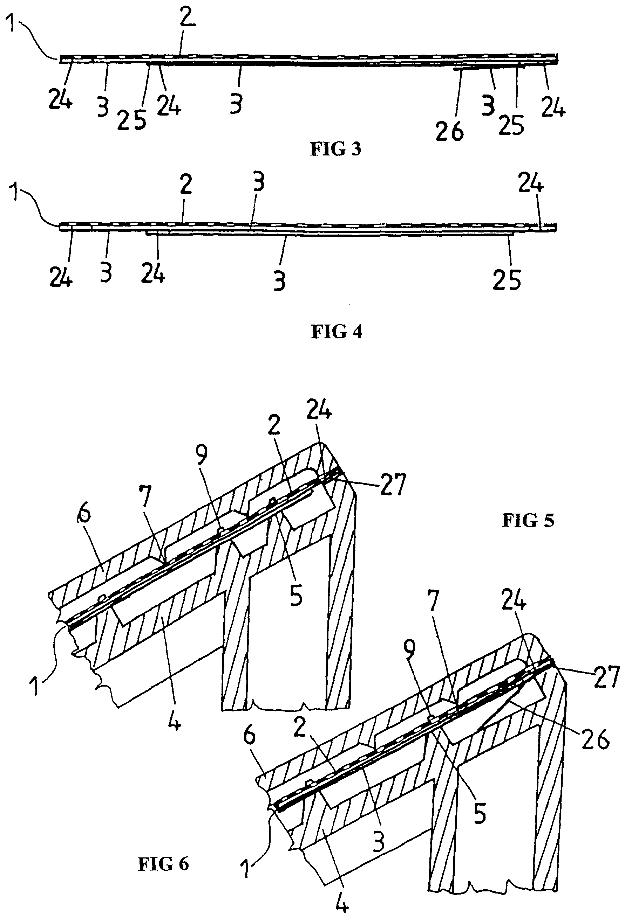

FIG. 3 shows that in case of an embodiment of the three-layered 1 measuring stripe, a solid structured 3 lower layer, made of celluloid base material, free of wood-fibres is stuck to both ends of the 2 upper layer, made of veil, comprising celluloid fibres with a 24 two side self-adhesive plastic tape. Then the 25 end of the third layer is placed at least 10 mm farther in from the end of the measuring stripe next to the reservoir. The 25 end of the third layer is also fixed on the also solid structured 3 lower layer, made of celluloid base material with also 24 two-side self-adhesive plastic tape. The length of the end of the 3 lower layer next to the 26 insertion holding end is min. 5 mm shorter than the full length of the measuring stripe. Finally a min. 10 mm long 26 insertion holding end is fixed on the end of the measuring stripe with a 24 two-side self-adhesive plastic tape.

The 1 measuring stripe, shown in FIG. 4 is essentially the same as the one in FIG. 3. The difference between them is only that there is no 26 insertion holding end in FIG. 4, as the end of the 3 tower layer is supported by the 5 distance piece, created on the 4 lower plane according to FIG. 5.

In case of the implementation as shown in FIG. 5 the 1 measuring stripe according to FIG. 4 is built in the device. Bending down of the end of the lowest, unfixed 3 lower layer of the measuring stripe is prevented by a 5 distance piece, particularly formed for this purpose. The end of the 1 measuring stripe fits in the 27 bracing slot.

In case of the implementation as shown in FIG. 6 the 1 measuring stripe according to FIG. 3 is built in the device. Bending down of the end of the 3 lower layer is prevented by the 26 insertion holding end which supports it.

According to the graph, shown in FIG. 7 there is a lineal relationship between the laboratory INR values (axis Y) and the blood clotting lengths, measured by the device (axis X) which can be expressed by $Y = 0.1393X - 3.3086$ regressive straight line, fitting on the measuring points, marked by black squares.

While assembling the device according to the invention, first we make the 15 INR value scale in the well-known way, then we fix it on the 20 plane, in parallel with the 1 measuring stripe.

While using the device, we fill up the 10 reservoir with blood sample, freshly taken from the finger pad, so that it entirely covers the 22 line showing blood level at the bottom of the 21 small sheet showing blood level. It means adding 6-8 drops of blood. While absorbing in the 1 measuring stripe, the blood sample begins to clot in it, thus setting limits to the extent process of the absorption of blood cells. In the end the absorption of blood cells is stopped as the absorbed, clotted blood blocks the way of the flowing blood cells.

You should be careful when filling up the reservoir with blood to make sure that it does not take longer than 2 minutes or else the measured INR values may differ from the laboratory ones more than acceptable. After taking blood you have to place the device on a horizontal surface and must not move it. The result of measuring can be read from the INR scale next to the blood column 20 minutes after taking blood.

## The Advantages of the IVD Device according to the invention

- It makes it possible to determine the INR value on the basis of coagulation in a rapid (within 20 min) and safe way,
- It is cheap, its structure is simple and its manufacturing does not require any complicated technical operations,
- There is no need to apply any reagents during measurements,
- It requires little blood sample to carry out measurements,
- It can be produced and traded as a mass product,
- It can reliably be used without any special expertise by assuring the process of coagulation without affecting the process artificially.

## Figure Legends

| | |
|---|---|
| 1 | measuring stripe |
| 2 | upper layer |
| 3 | lower layer |
| 4 | lower plane |
| 5 | distance piece |
| 6 | upper plane |
| 7 | distance mandrel |
| 8 | hinge |
| 9 | shoulder |
| 10 | reservoir |
| 11 | protruding piece |
| 12 | flange |
| 13 | lock pin |
| 14 | undercut |
| 15 | INR value scale |
| 16 | red zone |
| 17 | light blue zone |
| 18 | dark blue zone |
| 19 | green zone |
| 20 | plane |
| 21 | small sheet showing blood level |
| 22 | line showing blood level |
| 23 | evaluation part |
| 24 | two-side self adhesive plastic tape |
| 25 | end of third layer |
| 26 | insertion holding end |
| 27 | bracing slot |
| 28 | regressive straight line |

## Claims

1. A disposable device for home measuring of clotting capacity of a blood sample free of reagents, in particular of the INR value, with a blood absorbing material through measuring blood clotting distance, said device comprising

   - two planes, a lower plane (4) and an upper plane (6) being at least partially transparent, a reservoir (10), and an elongated measuring stripe (1) free of reagents and arranged to be brought into contact with the blood sample in the reservoir (10) attached to the lower plane (4), said measuring stripe (1) being braced between distance mandrels (7) and distance pieces (5) protruding from said planes, said distance mandrels (7) and distance pieces (5) locating nonoverlapping to one another; and
   - an expediently chosen evaluation part (23), supplied with an INR value scale (15), suitable for reading off the boundary line of the clot on the measuring stripe (1) in case of measuring particularly the INR value, wherein the measuring stripe (1) comprises at least two parts, such as an upper layer (2) and a lower layer (3),

      said upper layer (2) comprising at least one loose structured, fibrous absorbent material provided by e.g. one or more layers of veil, made of loose structured plant fibres or synthetic fibres or orderly, veil-like heap of their mixture or paper, made of veil-like celluloid fibres, synthetic veil, blotting paper, said lower layer (3) comprising at least one thicker, solid structured cardboard with a glossy and smooth surface, made of less absorbent celluloid base material, said two parts being joined together by sticking at their ends, wherein at least one further layer is joined to said two parts, to the lower layer (3) thereof, by sticking at their ends in such a way that one end of said further layer locates at least 10 mm farther from the end of the measuring stripe (1) being at the reservoir, while its other end is at least 3 mm shorter than the other end of said two parts, the free other end of said further layer being supported by a distant piece (5) or an insertion holding end (26) fixed to said other end of the measuring stripe so that the end of said further layer can slip in case of stretching.

2. The device as claimed in claim 1 wherein the upper plane (6) is transparent in at least one third of its length from the end opposite the reservoir (1).

**3.** The device as claimed in claim 1 or 2 wherein the distance pieces (5) protruding from the lower plane (4) are formed as shoulder-like distance pieces.

**4.** The device as claimed in any of claims 1-3 wherein the reservoir (10) is supplied with a small sheet showing blood level (21).

**5.** The device as claimed in claim 4 wherein the small sheet showing blood level (21) marks the amount of blood to be filled into the reservoir (10) with a line showing blood level (22).

**6.** The device as claimed in any of claims 1-5 wherein the ends of the distance mandrels (7) protruding from the upper plane (6) are formed point-like.

**7.** The device as claimed in any of claims 1-6 wherein

a) the upper layer/layers of the measuring stripe (l) is/are formed as loose structured veil(s) comprising uniformly distributed fibres, ironed together with a weight of 50-100 gr/m$^2$ and a thickness of < 0.5 mm,

b) the lower layer/layers of the measuring stripe (l) is/are formed as a solid structured, glossy and smooth surfaced, less absorbent carboard/carboards, made of celluloid basic material free of wood fibres with a weight of > 100 gr/m$^2$ and a thickness of 0.2-1 mm,

c) the upper layer/layers and the lower layer/layers are joined together at least at one end thereof, and

d) the width of said measuring stripe (l) is at least 1 mm.

**Patentansprüche**

**1.** Einwegvorrichtung für die reagenzienfreie Heimmessung der Gerinnungsfähigkeit einer Blutprobe, insbesondere des INR-Wertes, mit einem blutabsorbierenden Material durch Messung der Blutgerinnungsdistanz, wobei die Vorrichtung umfasst

- zwei Ebenen, eine untere Ebene (4) und eine obere Ebene (6), die mindestens teilweise transparent sind, ein Reservoir (10) und einen länglichen Messstreifen (1), der frei von Reagenzien ist und so angeordnet ist, dass er mit der Blutprobe in dem Reservoir (10), das an der unteren Ebene (4) angebracht ist, in Kontakt gebracht werden kann, wobei der Messstreifen (1) zwischen Abstandsdornen (7) und Abstandsstücken (5), die aus den Ebenen herausragen, eingespannt ist, wobei die Abstandsdorne (7) und Abstandsstücke (5) nicht überlappend zueinander angeordnet sind; und

- ein zweckmäßig gewähltes Auswerteteil (23), das mit einer INR-Wert-Skala (15) versehen ist, die zum Ablesen der Grenzlinie des Gerinnsels auf dem Messstreifen (1) bei der Messung insbesondere des INR-Wertes geeignet ist, wobei der Messstreifen (1) mindestens zwei Teile, wie etwa eine obere Schicht (2) und eine untere Schicht (3), umfasst,

wobei die obere Schicht (2) mindestens ein locker strukturiertes, faseriges Absorptionsmaterial umfasst, das z. B. von einer oder mehreren Schichten eines Schleiers bereitgestellt wird, der aus locker strukturierten Pflanzenfasern oder synthetischen Fasern oder einem geordneten, schleierartigen Haufen ihrer Mischung oder Papier besteht, das aus schleierartigen Zelluloidfasern, synthetischem Schleier oder Löschpapier besteht,

wobei die untere Schicht (3) mindestens eine dickere, fest strukturierte Pappe mit einer glänzenden und glatten Oberfläche umfasst, der aus weniger stark absorbierendem Zelluloid-Grundmaterial besteht, wobei die beiden Teile durch Verkleben an ihren Enden miteinander verbunden sind, wobei

mindestens eine weitere Schicht mit den beiden Teilen, mit der unteren Schicht (3) davon, durch Verkleben an ihren Enden so verbunden ist, dass ein Ende der weiteren Schicht mindestens 10 mm weiter von dem am Reservoir befindlichen Ende des Messstreifens (1) liegt, während ihr anderes Ende mindestens 3 mm kürzer ist als das andere Ende der beiden Teile, wobei das freie andere Ende der weiteren Schicht durch ein entferntes Stück (5) oder ein Einsteck-Halteende (26), das an dem anderen Ende des Messstreifens befestigt ist, gehalten wird, so dass das Ende der weiteren Schicht im Falle einer Dehnung verrutschen kann.

**2.** Vorrichtung nach Anspruch 1, wobei die obere Ebene (6) auf mindestens einem Drittel ihrer Länge von dem dem Reservoir (1) gegenüberliegenden Ende transparent ist.

**3.** Vorrichtung nach Anspruch 1 oder 2, wobei die aus der unteren Ebene (4) herausragenden Abstandsstücke (5) als schulterartige Abstandsstücke ausgebildet sind.

**4.** Vorrichtung nach einem der Ansprüche 1-3, wobei der Behälter (10) mit einem kleinen Blatt versehen ist, das den Blutspiegel (21) anzeigt.

**5.** Vorrichtung nach Anspruch 4, wobei das kleine Blatt, das den Blutspiegel (21) anzeigt, die in das Reservoir (10) einzufüllende Blutmenge mit einer Linie markiert, die den Blutspiegel (22) anzeigt.

**6.** Vorrichtung nach einem der Ansprüche 1-5, wobei die aus der oberen Ebene (6) herausragenden Enden der Abstandsdorne (7) punktartig ausgebildet sind.

**7.** Vorrichtung nach einem der Ansprüche 1-6, wobei

a) die obere(n) Schicht(en) des Messstreifens (1) als locker strukturierte(r) Schleier ausgebildet ist/sind, der/die gleichmäßig verteilte Fasern umfasst/umfassen, die mit einem Gewicht von 50-100 gr/m$^2$ und einer Dicke von < 0,5 mm zusammengebügelt sind,
b) die untere(n) Schicht(en) des Messstreifens (1) als eine fest strukturierte, weniger stark absorbierende Pappe(n) mit glänzender und glatter Oberfläche ausgebildet ist/sind, die aus holzfaserfreiem Zelluloid-Grundmaterial mit einem Gewicht von 100 gr/m$^2$ und einer Dicke von 0,2-1 mm besteht/bestehen,
c) die obere(n) Schicht(en) und die untere(n) Schicht(en) mindestens an einem Ende miteinander verbunden sind, und
d) die Breite des Messstreifens (1) mindestens 1 mm beträgt.

## Revendications

**1.** Dispositif jetable pour mesurer à domicile la capacité de coagulation d'un échantillon de sang exempt de réactifs, en particulier la valeur INR, avec un matériau absorbant le sang en mesurant la distance de coagulation sanguine, ledit dispositif comprenant

- deux plans, un plan inférieur (4) et un plan supérieur (6) qui sont au moins partiellement transparents, un réservoir (10) et une bandelette de mesure allongée (1) exempte de réactifs et agencée pour être mise en contact avec l'échantillon de sang dans le réservoir (10) fixé au plan inférieur (4), ladite bandelette de mesure (1) étant soutenue entre des mandrins d'espacement (7) et des pièces d'espacement (5) faisant saillie à partir desdits plans, lesdits mandrins d'espacement (7) et lesdites pièces d'espacement (5) se plaçant sans se chevaucher les uns par rapport aux autres ; et
- un élément d'évaluation (23) judicieusement choisi, doté d'une échelle de valeur INR (15), adapté à la lecture de la ligne limite du caillot sur la bandelette de mesure (1) en cas de mesure notamment de la valeur INR, dans lequel la bandelette de mesure (1) comprend au moins deux parties, telles qu'une couche supérieure (2) et une couche inférieure (3),

ladite couche supérieure (2) comprenant au moins un matériau absorbant fibreux à structure lâche constitué, par exemple, d'une ou de plusieurs couches de voile, composé de fibres végétales ou de fibres synthétiques à structure lâche, ou d'un tas ordonné de type voile de leur mélange ou de papier, composé de fibres de celluloïd de type voile, de voile synthétique, de papier buvard,
ladite couche inférieure (3) comprenant au moins un carton structuré plus épais et solide présentant une surface brillante et lisse, composé d'un matériau de base celluloïd moins absorbant, lesdites deux parties étant reliées ensemble par collage au niveau de leurs extrémités, dans lequel
au moins une couche supplémentaire est reliée auxdites deux parties, à la couche inférieure (3) de celles-ci, par collage au niveau de leurs extrémités de telle sorte qu'une extrémité de ladite couche supplémentaire se trouve à au moins 10 mm plus loin de l'extrémité de la bandelette de mesure (1) qui est au niveau du réservoir, tandis que son autre extrémité est au moins 3 mm plus courte que l'autre extrémité desdites deux parties, l'autre extrémité libre de ladite couche supplémentaire étant supportée par une pièce d'espacement (5) ou une extrémité de maintien d'insertion ( 26) fixée à ladite autre extrémité de la bandelette de mesure de telle sorte que l'extrémité de ladite couche supplémentaire puisse glisser en cas d'étirement.

**2.** Dispositif selon la revendication 1, dans lequel le plan supérieur (6) est transparent sur au moins un tiers de sa longueur à partir de l'extrémité opposée au réservoir (1).

**3.** Dispositif selon la revendication 1 ou 2, dans lequel les pièces d'espacement (5) faisant saillie à partir du plan inférieur (4) sont formées sous la forme de pièces d'espacement en forme d'épaulement.

**4.** Dispositif selon l'une quelconque des revendications 1-3, dans lequel le réservoir (10) est muni d'une petite feuille indiquant un niveau de sang (21).

**5.** Dispositif selon la revendication 4, dans lequel la petite feuille indiquant un niveau de sang (21) marque la quantité de sang à verser dans le réservoir (10) avec une ligne indiquant un niveau de sang (22).

**6.** Dispositif selon l'une quelconque des revendications 1-5, dans lequel les extrémités des mandrins d'espacement (7) faisant saillie à partir du plan supérieur (6) sont formées en forme de point.

**7.** Dispositif selon l'une quelconque des revendications 1-6, dans lequel

a) la ou les couches supérieures de la bandelette de mesure (1) sont formées sous la forme d'un ou de plusieurs voiles à structure lâche comprenant des fibres uniformément réparties, repassées ensemble avec un poids de 50-100 gr/m$^2$ et une épaisseur < 0,5 mm,

b) la ou les couches inférieures de la bandelette de mesure (1) sont formées sous la forme d'un ou de plusieurs cartons moins absorbant à surface structurée solide, brillante et lisse, composés d'un matériau de base celluloïd sans fibres de bois avec un poids > 100 gr/m$^2$ et une épaisseur de 0,2-1 mm,

c) la ou les couches supérieures et la ou les couches inférieures sont réunies au moins au niveau d'une extrémité de celles-ci, et

d) la largeur de ladite bandelette de mesure (1) est au moins 1 mm.

FIG 1

**FIG 2**

FIG 3

FIG 4

FIG 5

FIG 6

FIG 7

Y axis: INR a laboratóriumban

X axis: Blood absorption (mm)

y = 0,1393x - 3,3086

28

■ measurements

EP 2 932 261 B1

13

## REFERENCES CITED IN THE DESCRIPTION

### Patent documents cited in the description

- US 6759009 B
- WO 9406007 A
- WO 9832389 A1
- WO 9600395 A
- US 6759009 A
- US 3951606 A